# EUROPEAN PATENT APPLICATION

(11) **EP 3 929 183 A1**
(43) Date of publication of application: **29.12.2021**
(21) Application number: 20760026.3
(22) Date of filing: 18.02.2020
(51) Int. Cl.: C07D 317/46, A61K 38/04, A61P 25/16

(54) **METHOD FOR SYNTHESIZING DOPA OLIGOPEPTIDE INTERMEDIATE AND USE, COMPOSITION AND PREPARATION THEREOF**

(30) Priority: 18.02.2019 CN 201910119642
(71) Applicant: Hainan University, Hainan 570228 (CN)
(72) Inventor: LIU, Zhongqiang, Haikou, Hainan 570228 (CN); ZHANG, Xiaoyan, Haikou, Hainan 570228 (CN); HU, Bihuang, Haikou, Hainan 570228 (CN); XIONG, Feng, Haikou, Hainan 570228 (CN); FAN, Lixia, Haikou, Hainan 570228 (CN); HE, Yanmiao, Haikou, Hainan 570228 (CN); NING, Donghua, Haikou, Hainan 570228 (CN); FU, Kaizhong, Haikou, Hainan 570228 (CN); CHEN, Sikang, Haikou, Hainan 570228 (CN); ZHONG, Shibo, Haikou, Hainan 570228 (CN); WAN, Ying, Haikou, Hainan 570228 (CN); YAO, Rui, Haikou, Hainan 570228 (CN); LI, Mengdi, Haikou, Hainan 570228 (CN)
(74) Representative: Sach, Greg Robert
(86) International application number: PCT/CN2020/075639
(87) International publication number: WO 2020/169012

(57) **Abstract**

This invention belongs to the medicine field and discloses a synthetic method of L-DOPA-containing oligopeptide derivatives and pharmaceutical applications. Specifically, it includes an improved synthesis of ketal-protected L-DOPA intermediates, the synthesis of fatty acid conjugates with L-DOPA-containing dipeptides using the above key intermediates. The said ketal-protected intermediate can be synthesized in two steps with high yields, eliminating the two steps of protection and deprotection of a carboxyl group used in the previous art. This intermediate was applied to synthesize many end products, and among them, FDD-16, FDD-18 and FDD-14 can form gels in a wide range of concentrations, which exhibit good stabilities and long half-lives and are promising to be developed into anti-parkinsonism drugs with high bioavailability and long-term controlled-release effect.

## Description

### Technical Field

This invention belongs to the field of medicine and involves an improved method of ketal-protected L-DOPA intermediates and their products, applications and combinations. In particular, it includes the preparation of fatty acid complexes containing L-DOPA dipeptides and the design of prodrugs against Parkinson's disease by using ketal protected L-DOPA intermediates and fatty acids.

### Background

Parkinson's disease (PD) is the second major neurodegenerative disease which seriously threatens human health. For more than half a century, L-DOPA has been the gold standard for treating PD. Many novel anti-Parkinsonism drugs are developed through modification of L-DOPA. Meanwhile, L-DOPA is a rare natural amino acid and L-DOPA-containing oligopeptides, polypeptides or polymer materials have a wide range of pharmaceutical applications.

However, L-DOPA have many shortcomings and disadvantages. For example, the catechol group of L-DOPA can be easily oxidized by oxygen in the air or by other oxidants. Therefore, during the synthesis of L-DOPA derivatives, appropriate protective groups (PG) are needed to shield the catechol. Extensive literature reports demonstrated that acetonide is a perfectly suitable protective group for catechol.

The Fmoc-based solid-phase peptide synthesis (SPPS) method has become a routine choice for the preparation of peptides because of strong applicability, easy operation, low price and wide application. Fmoc-DOPA(Acetonide)-OH is a key intermediate for the solid-phase synthesis of L-DOPA-containing oligopeptides, polypeptides and complexes. L-DOPA has low bioavailability (1-3%), short time of effective blood drug concentration (50min), and toxic side-effects when taken in large quantities for a long time. Therefore, various measures have been taken to reduce the daily dose and improve the bioavailability of L-DOPA, such as making compound preparations containing metabolic enzyme inhibitors, such as Metobar, Sinemet (the half-life of L-DOPA was about 90min) and Stalevo (135min). Oligopeptide derivatives containing L-DOPA provide certain degrees of protection for L-DOPA, thus reducing the metabolism of L-DOPA in the digestive tract and blood circulation. Oligopeptide Transporters on the small intestine wall allow oligopeptides absorbed directly, thus improving the absorption rate of L-DOPA. It has been found that L-DOPA-containing linear dipeptides formed with different amino acid exhibited varied anti-degradation abilities in rat liver homogenate. For example, the half-life of H-DOPA-Asp-OH is about 174min while the half-life of H-DOPA-Met-OH is only 16min.

Proteins, lipids and carbohydrates are three essential nutrients for the body. PD patients are prone to malnutrition due to long-term medication, which in turn impairs the effectiveness of drug treatment. Therefore, how to ensure a balanced nutrition for PD patients is very important. Studies demonstrated that oligopeptide transporters absorb substrates rapidly, so if L-DOPA-containing oligopeptides are administrated, they can provide PD patients with both drugs and protein supplements. Fatty acids and their derivatives can be absorbed as chylomicron or by free diffusion through the small intestinal chorion. Patent WO2006119758 A2 and WO2010103273 A3 fabricated a series of complexes of fatty acid and L-DOPA. Their experiments showed that dopamine concentrations and retention times in rat brains were better than those in the control group. However, they did not test or mentioned complexes formed from fatty acids and L-DOPA oligopeptides. Though Linear dipeptides are the smallest oligopeptides, they possess quite different chemical or physical properties, comparing with single amino acid. Some dipeptides can self-assemble into ordered structures. Dr. Lin Shuwei from Yang's research group pointed out that lipodipeptides and lipo-tripeptides can self-assemble into gels in aqueous or organic phases. Administration of L-DOPA-containing lipopeptides can provided patients with both L-DOPA and supplements of fatty acids and other amino acids.

Oral administration of L-DOPA preparations causes great fluctuation of blood L-DOPA concentrations in a day, which goes against a stable control of Parkinson's symptoms. In addition, such impulsive stimulation of dopaminergic neurons is the main cause of their further apoptosis. Late PD patients are in incapacitation with poor mobility and uncontrolled vomiting, which impairs oral medication efficacy. Therefore, AbbVie's enteral gel, Duopa, has a great advantage. It was formed by dispersing L-DOPA and Carbi-DOPA(4:1) with carboxymethyl cellulose (sodium) in water. Then, an injection pump was used to deliver the gel through a tube buried across the stomach wall into the small intestine (in 16h ), ensuring a stable blood L-DOPA concentration.

Therefore, it is possible to form new compound preparations or gel preparations by replacing L-DOPA in Medopar or Duopa with L-DOPA-containing oligopeptides or lipooligopeptides and obtain a new formula with higher bioavailability and steadier blood concentration.

Because the catechol group of L-DOPA can be easily oxidized, a suitable protective group (PG) is needed to protect the side chain in the chemical derivation of L-DOPA. To produce L-DOPA-containing oligopeptides using Fmoc-based solid phase peptide synthesis (SPPS) protocol, Fmoc-DOPA(Acetonide)-OH (Compound I) is a key intermediate.

The US patent US8227628 revealed that acetone protection of L-DOPA is accompanied with a competitive Pictet-Spengler reaction and It presented two systematic and universal synthetic methods for Fmoc-DOPA(Acetonide)-OH. They can be applied to prepare most acetal/ketal-protected L-DOPA and dopamine derivatives. They have the following advantages: The yield of each step is relatively high; product separation and purification are quite simple, especially for the fully-protected intermediates. For example, Tfa-DOPA(Acetonide)-OMe is structurally very stable and easy to be purified through simple recrystallization. It can be readily converted into a variety of useful intermediates. There are also some disadvantages: the universal methods are set to prepare various ketal-protected L-DOPA derivatives and thus are not designed specifically for the synthesis of one target ketal-protected compound. For example, it takes 4-5 steps to prepare the most important acetonide derivative Fmoc-DOPA(Acetonide)-OH, which involves the replacement of the amino protecting groups: Firstly, Tfa- or Phth- is used to protect the amino group of L-DOPA and methyl ester is used to mask the carboxyl group of L-DOPA; Then, using TsOH as a catalyst, 2,2-dimethoxypropane (DMP) was refluxed with the intermediate in benzene to form acetonide; Finally, Phth- or Tfa- was removed with base and Fmoc-OSu was employed to install Fmoc protecting group on the amino group to form the target product.

Patent US8227628 stated that to exploit hydrogenation deprotection that is orthogonal to acid and base protection/deprotection, the carboxyl of L-DOPA can be protected as a benzyl ester. Patent WO2013/168021 A1 applied a similar acetonide cyclization strategy (TsOH as a catalyst, refluxing in benzene, calcium chloride to adsorb MeOH) to produce acetonide-protected Carbi-DOPA derivatives for synthesizing potential drugs for degenerative neurological diseases.

Based on the US patent application US2010/0087622A1, a Chinese patent CN102718739A raised a two-step synthetic method for Fmoc-DOPA(Acetonide)-OH. The apparent merit of this method is that the synthesis needs only two steps to complete: the first step is to synthesize Fmoc-DOPA-OH intermediate, then the second step is to install acetonide by refluxing with DMP in THF in the presence of a pyridine salt of TsOH (TsOH-Pyr) for extended time. The shortcomings of this method include: lack of proper techniques to shift reaction equilibrium, partial completion of acetonide cyclization, presence of a large amount of unreacted Fmoc-DOPA-OH reactant, good to acceptable yields, and substantial difficulties in product separation and purification.

The acetal/ketal formation between catechol and aldehyde/ketone is a reversible reaction. The equilibrium constant is usually low and the reaction rate difference between the forward and the backward reaction is not large. Therefore, it is very important to timely and effectively remove the byproduct water /MeOH from the reaction system, which helps to improve product yields and reduce purification difficulties. Comparing the two-component phase diagrams of water-benzene, MeOH-benzene, water-THF and MeOH-THF, benzene performs better than THF in carrying MeOH/ water out of the liquid phase. Even tiny percentages of MeOH/water is present in benzene, they can be quickly distilled out from the reaction system, thus reducing the residual reactant to minimum and improving the target product yield. The reagents commonly used to install acetonide to catechol include acetone (least reactivity); DMP (proper reactivity); 2-methoxypropene (2MT, highest reactivity). In addition, DMP can react with water to generate acetone and MeOH, so even using DMP as an acetonide cyclizing reagent it requires to remove water from the system as much as possible. Anhydrous calcium chloride can adsorb both water and MeOH, so it is the best adsorbent for byproducts removal in this reaction.

Since there are so many shortcomings in the existing technology, it is still a great challenge to find a preparation method suitable for industrial production of L-DOPA-containing pharmaceutical intermediates with mild reaction conditions, simple operation, high yields, high purities and low cost.

### Disclosure of the Invention

One purpose of this invention is to provide a synthetic method for key pharmaceutical intermediates, namely, a method for key intermediates (such as Fmoc-DOPA (Acetonide) -OH, Fmoc-DOPA (cyclohexanonide) -OH) to produce L-DOPA-containing oligopeptides. The advantages of this invention lie in that the key intermediates can be synthesized in only two steps with high yields and that the presented method is suitable for industrial production.

Another purpose of this invention is to explore a synthetic method for anti-Parkinson's disease prodrugs with controlled slow release properties, in the form of conjugates of fatty acids and L-DOPA-containing dipeptides.

A further purpose of this invention is to provide drugability studies of complexes containing fatty acids and L-DOPA or L-DOPA dipeptides.

Specifically, the technical scheme of this invention is as follows.

A synthetic method for key intermediates to produce L-DOPA-containing oligopeptides, using compound II as the starting material, using organic solvent *α* as the co-solvent, under the catalytic action of acid *b* to obtain compound I. The reaction equation is as follows.

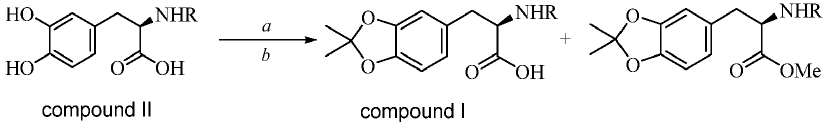

Organic solvent *a* and compound II are added to the reaction solvent, after refluxing under inert gas atmosphere the cyclizing reagent and strong acid *b* are added, and then absorbents are added to adsorb water and volatile byproducts. Finally, Fmoc-DOPA(Acetonide) -OH was obtained, namely compound I.

The mentioned organic solvent *a* is an anhydrous solvent of acetone, benzene, toluene, xylene, chlorobenzene, dichlorobenzene, isopropanol, diethyl ether, methyl butanone, methyl isobutanone, or pyridine. A mixture of anhydrous acetone and anhydrous benzene is preferred.

The mentioned strong acid b is one or more of TsOH, camphorsulfonic acid, phenylhexacarboxylic acid, hydrogen halides, trifluoroacetic acid, acetic acid, and strong acid ion exchange resins. TsOH is preferred.

The mentioned cyclizing reagent is one of acetone, 2,2-dimethoxypropane (DMP), 2-methoxypropene (2MT), cyclopentanone, cyclohexanone, diphenylketone, other ketone or aldehyde derivatives or a combination thereof. DMP or cyclohexanone is preferred.

The mentioned amino protecting group R- includes but is not limited to Phth -, TCP-, Dts-, Tfa-, Ac-, Aloc-, Meco2-, EtCO2-, Boc-, Fmoc-, Teoc-, Troc -, SES-, and Tr-. Fmoc- is preferred and adopted as an illustrating example.

Anhydrous calcium chloride (CaCh), which rapidly absorbs water and MeOH, is the main absorbent of volatile byproducts. It can be used with or without other absorbents, including but not limited to anhydrous magnesium sulfate, anhydrous sodium sulfate, molecular sieves. The apparatus for holding the absorbents may be a thimble in a Soxhlet extractor or a constant pressure dropping funnel with fritted-glass. Volatile byproducts can also be removed by atmospheric or vacuum distillation, with a fluid pump to slowly replenish the distilled-out solvents and cyclizing reagents. A Soxhlet extractor with the thimble filled with anhydrous calcium chloride is preferred.

The molar ratio of compound II to DMP is 0.8-20, preferably 2.5; The molar ratio of compound II to cyclohexanone is 1-50, preferably 10. The reaction temperature was controlled at the reflux temperature of the solvents, ranging from 80 °C to 120°C.

The preparation process of compound II is as follows.

(1) Add Na₂B₄O₇·10H₂O and water into a 1000ml three-neck flask, stir well.

(2) After passing inert gas such as nitrogen or argon for 30min, L-DOPA and Na₂CO₃ are added, Fmoc-OSu dissolved in THF is added dropwise, and stir well. Adjust the solution with 2N HCl to pH =3 , add some Na₂S₂O₃, reduce the solvent by rotary evaporation, then extract with EtOAc, take the organic layer, which is washed with water, dried over anhydrous magnesium sulfate, filtered, and subjected to rotary evaporation to a small amount. The concentrated organic residue is added with petroleum ether, and compound II is precipitated out.

Compared with the prior art, the technical effects of this invention are as follows:

Fmoc-DOPA (Acetonide) -OH is obtained in two steps, avoiding the two reductant steps of carboxyl protection and deprotection reactions.

(1) Since Fmoc-DOPA-OH has a bad solubility in benzene, anhydrous acetone and anhydrous benzene are mixed to serve as solvents. It makes good use of the ability of benzene to carry water and MeOH out through azeotropic distillation and the ability of acetone to largely increase the reactant solubilities in the reaction solution.

(2) By applying TsOH, camphorsulfonic acid and other strong acids as catalysts, instead of weakly acidic 2,4,6-trimethylpyridine *p*-toluenesulfonate, the reaction rate is inreased, the reaction time is shortened, and generation of byproducts is reduced.

(3) By Adopting CaCl₂ to absorb water and MeOH generated in the reaction system, the reactant Fmoc-DOPA-OH is completely consumed and thus does not contaminate the target product, rendering no difficulty for product separation. A main byproduct generated through this method was separated and full characterized. It was identified as Fmoc-DOPA (Acetonide) -OMe, which does not interfere with SPPS and does not need to be removed, thus further diminishing product purification difficulties.

(4) This invention has the advantages of two synthetic steps, high yield, low cost, mild reaction conditions, simple operation, no need for high pressure, high temperature or other harsh reaction conditions, no requirements for multiple times recrystallizations or other operations.

Another purpose of this invention is that by applying compound I to synthesize new intermediates or end products, novel slow-releasing anti-Parkinsonism drug candidates are obtained, specifically including L-DOPA containing oligopeptides, conjugates of fatty acids with L-DOPA or L-DOPA-containing oligopeptides.

One product is a conjugate of fatty acid and L-DOPA.

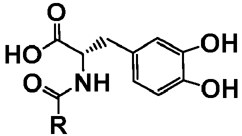

Wherein, R represents chains of linear or branched fatty acids, including saturated or unsaturated fatty acids, such as palmitic acid, tetradecanoic acid, lauric acid, stearic acid, linoleic acid, oleic acid, linolenic acid, docosahexaenoic acid. One of palmitic acid, stearic acid, or tetradecanoic acid is more preferred.

Specific synthetic method is as follows:
a) Chlorotrityl chloride (CTC) resin is activated by adding SOCl₂ / DCM, and then washed with DCM.
b) Fmoc-DOPA(Acetonide)-OH is dissolved in DCM, followed by addition of diisopropylethylamine (DIEA). The mixture is added to a SPPS tube.
c) DIEA /MeOH/DCM is applied to cap the remaining reactive sites on the resin, and then Fmoc is removed with 20% 4-methylpiperidine in DMF.
d) Stearyl chloride is dissolved in an appropriate amount of DCM, followed by addition of DIEA. The mixture is poured into the SPPS tube, shaking for 5-16h.
e) After the reaction is completed as verified by ninhydrin test, the resin is washed and eluted with 2% TFA/DCM solution, and a conjugate of L-DOPA and stearic acid is obtained in the protected form.
f) When in need, a sample of the stearic acid and L-DOPA conjugate is prepared by removing acetonide protection with TFA/(Triisopropylsilane)TIS/H₂O (95/2.5/2.5).

### Another product is a conjugate of fatty acid and L-DOPA-containing dipeptide

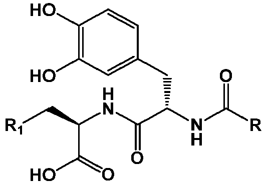

Wherein, R represents chains of linear or branched fatty acids of C₁₂ ~ C₃₀, including saturated or unsaturated fatty acids, such as lauric acid, palmitic acid, tetradecanoic acid, lauric acid, stearic acid, linoleic acid, oleic acid, linolenic acid and docosahexaenoic acid; more preferably one of palmitic acid, stearic acid and tetradecanoic acid. R₁ is the side chain of L-type or D-type amino acids with good compatibility with human body, including but not limited to 20 common amino acids, beta-alanine, taurine and citrulline; Asp is preferred. The position of L-DOPA can be located at the *N*-terminal or C-terminal of the dipeptides. The illustrated structure takes the *N*-terminal as an example.

The target products are prepared by Fmoc-based SPPS protocol and the synthesis scheme is shown in the figure below.

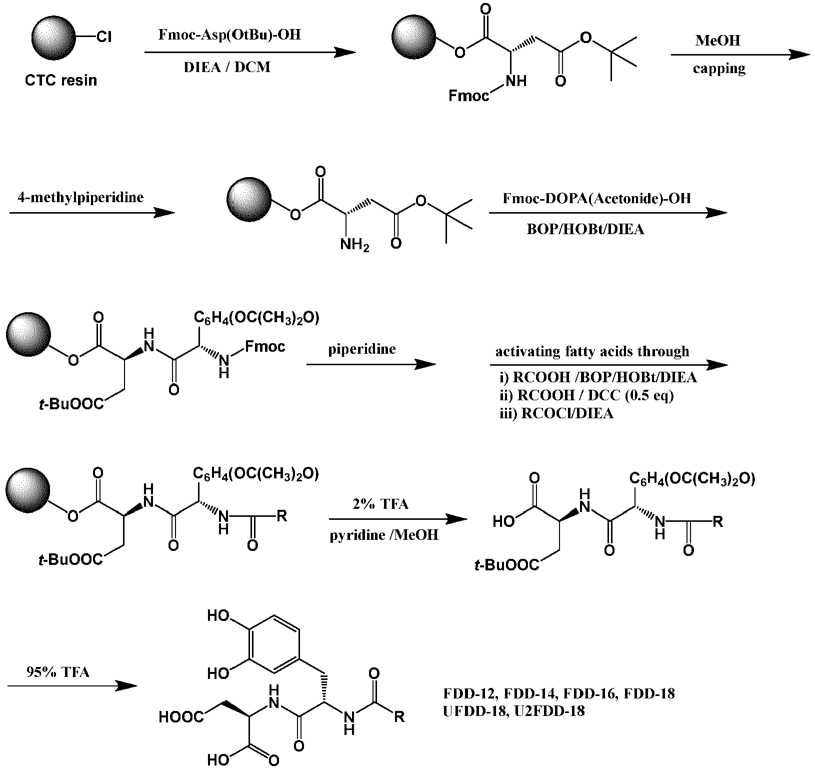

The method is summarized as follows:
It takes CTC resin as a solid-supporting material, Fmoc-DOPA(Acetonide)-OH and Fmoc-Asp(OtBu)-OH as reactants, 4-methylpiperidine as Fmoc- deprotecting reagent and the acyl chloride, anhydride or activated ester (BOP / HOBt) as the activated from of fatty acids. The synthesized conjugates of fatty acids with L-DOPA-containing dipeptides are cut off from the resin with 2% TFA, and the obtained intermediate products are in the side-chain protected forms. When in need, the side chain protection is quickly removed with 95% TFA and the target conjugates of fatty acids and L-DOPA-containing dipeptides are obtained in unprotected forms.

Based on the above technology, linear or branched fatty acids of C12 ~ C30 are applied to synthesize the target products, specifically, as follows.

### Form 1: Abbreviations of fatty acid conjugates Containing L-DOPA

| Abbreviations fatty acids | Structures | activating methods for |
|---|---|---|
| FD-12 | conjugates of lauric acid & L-DOPA | Bought lauryl chloride |
| FD-18 | conj. of stearic acid & L-DOPA | Bought stearyl chloride |
| FDD-16 | conj. of palmitic acid & L-DOPA-L-Asp | BOP/HOBt/DIEA |
| FDD-14 | conj. of C14 fatty acid & L-DOPA-L-Asp | BOP/HOBt/DIEA |
| FDD-12 | conj. of lauric acid & L-DOPA-L-Asp | Bought lauryl chloride |
| FDD-18 | conj. of stearic acid & L-DOPA-L-Asp | Bought stearyl chloride |
| UFDD-18 | conj. of oleic acid & L-DOPA-L-Asp | BOP + DCC-anhydride |
| U2FDD-18 | conj. of linolenic acid & L-DOPA-L-Asp | BOP + DCC-anhydride |

Another purpose of this invention is to provide a pharmaceutical composition comprising fatty acid derivatives, fatty acid conjugates, new intermediates or products synthesized with compound I, or pharmaceutically acceptable salts thereof, with or without one or more pharmaceutically acceptable excipients.

Another purpose of this invention is to provide a drug combination model for supplementing nutrition and simultaneously providing L-DOPA drug for Parkinson's patients. It comprises any combination of L-DOPA containing oligopeptides, fatty acids, L-DOPA complexes and lipo-oligopeptides. Preferably, it comprises any combination of compound I, L-DOPA dipeptides, fatty acids and L-DOPA complexes, or intermediates or products synthesized from fatty acids and L-DOPA oligopeptide complexes, compound I, or pharmaceutically acceptable salts thereof, with or without one or more pharmaceutically acceptable excipients.

Another purpose of this invention is to provide an anti-parkinsonism preparation, which comprises L-DOPA-containing oligopeptides, lipo-oligopeptides, with or without L-DOPA metabolic enzyme inhibitors, with or without medicinal polymer materials.

The preparation is further selected in the form of a gel, an injection or an oral agent.

Compared with the prior art, the invention has the following significant advantages:
Through gelation test, it demonstrates that it is a new idea for designing drugs to treat Parkinson's disease. Among the end products, FDD-12 doses not form gels; FDD-16, FDD-18 and FDD-14 can form stable gels. FDD-16 and FDD-14 can form gels in a wide range of concentrations, which is suitable for further pharmaceutical preparation tests.

### Figures Attached:

Fig. 1: characteristic diagram of gel formation with FDD-16.
Fig. 2: the electron microscope (SEM) diagram of the gel formed with FDD-18
Fig. 3: the electron microscope (SEM) diagram of the gel formed with FDD-14

### Specific Embodiments

To verify the feasibility of the technical scheme of this invention, inventors have carried out research on the synthetic methods of key intermediates and the end products as well as the characterization techniques. It should be noted that the synthetic methods and detection techniques of the key intermediates and end products of this invention are only representative, and other synthetic methods, end products and detection techniques included in this invention are not exhausted herein due to space limitations.

### Example 1: Synthesis of Fmoc-DOPA(cyclohexanonide)-OH as follows

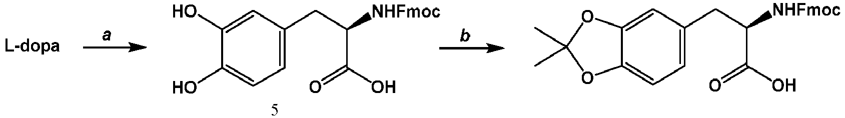

*a.* An amount of 14.3g Na₂B₄O₇·10H₂O (37.5mmol), 200ml water and a magnetic stirring bar were added into a 1000ml three-neck flask. After passing argon for 30min, 14.8g L-DOPA (75mmol) and 8.0g (75mmol) Na₂CO₃ were added, followed by addition of Fmoc-OSu (27.8g, 90mmol) in 200 ml THF with a dropping funnel. After stirring for 12 hours, the solution was adjusted to pH = 3 with 2N HCl solution, followed by addition of 10-20g Na₂S₂O₃. The mixture was reduced with rotary evaporation, and then extracted with EtOAc. The organic layer was washed with water, dried over anhydrous magnesium sulfate. After filtration, the filtrate was reduced to a small amount with rotary evaporation, followed by addition of petroleum ether to give chemical **5** (white powder, 28.9g, 91%). HRMS: [M+H]⁺ Calcd. 420.1442, Found 420.1448.

To screen the optimal conditions for b-step reaction, probing tests were carried out with a 100ml two-neck flask, using 2.1g (5mmol) chemical **1** as the reactant, CaCl₂ as the adsorbent and HPLC as the detection means. The optimal reaction conditions were screened for: major solvents, co-solvents, acetonide-providing reagents, DMP molar ratios, reaction temperatures, reaction times and catalysts. It found that among the tested solvents of benzene, toluene, acetone, THF and DMF, benzene gave the best result (byproduct impurity **6**), followed by toluene (byproduct impurity **7**). Using THF or DMF as the major solvents, only tiny amount of Compound **I** was detected. Due to the low solubility of chemical **5** in benzene, it was found that after addition of acetone as cosolvent, the yield of Compound **I** was almost doubled, while no such increase was observed when using THF or DMF as the cosolvent. In terms of catalyst screening, it was found that strong acids with low oxidizing abilities performed better: TsOH > camphor sulfonic acid >TFA> HOAc. In terms of acetonide-providing reagent screening: the reactivity of acetone was too low and DMP was quite suitable; 2MT was too reactive and it converted the carboxyl group of L-DOPA into a methyl ester (impurity **8**) even at low temperature. In terms of the molar ratio screening of DMP to chemical 5: in case the ratio is less than 2, the reaction cannot complete; in case the ratio was 10 or higher, significant percentages of L-DOPA methyl ester were formed; the best choice of the molar ratio was 2.5.

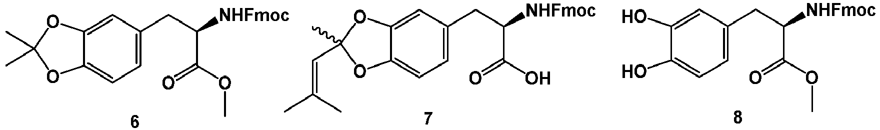

*b.* To a 100ml two-neck flask, were added 2.1g (5mmol) of chemical **5**, 5ml of anhydrous acetone and 70ml of anhydrous benzene. After heating and refluxing under argon for 15 minutes, 1.5ml (12.5mmol) of DMP and 20mg TsOH were added. The byproducts H₂O / MeOH generated in the reaction system were removed with anhydrous CaCl₂ (filled in a Soxhlet extractor or a constant-pressure dropping funnel with fritted glass). The reaction process was monitored with ferric chloride test, and it took about 1-2h to complete. After cooling, the reaction mixture was filtered through a short silica-gel column, which was washed with DCM / EtOAc. The combined filtrate was subjected to rotary evaporation to give a light-yellow solid, which was recrystallized in EtOAc / petroleum ether to produce target chemical **4** (2.0g, 89%).

Chemical 6: HRMS [M+H]⁺ Calcd. 420.1442, Found 420.1448. ¹HNMR (400 MHz, DMSO-d*₆*) δ 7.89(s, 1H), 7.87(s, 1H), 7.66-7.63 (m, 2H), 7.43-7.39(m, 2H), 7.34-7.28(m, 2H), 6.67(d, J=2.1Hz, 1H), 6.63(d, J=8.0Hz, 1H), 6.52(dd, J=8.0, 2.1Hz, 1H), 4.20(m, 3H), 4.08(m, 1H), 2.90(dd, J=13.8, 4.6Hz, 1H), 2.70(dd, J=13.8, 10.2Hz, 1H). ¹³CNMR δ 173.56, 155.97, 144.93, 143.82, 143.77, 140.67, 128.71, 127.64, 127.12, 125.37, 125.28, 120.09, 119.86, 116.47, 115.33, 65.67, 55.95, 46.59, 36.04.

Chemical 7: HRMS [M+H]⁺ Calcd. 460.1755, Found 460.1750. ¹HNMR (400 MHz, DMSO-d*₆*) δ 7.88(s, 1H), 7.86(s, 1H), 7.64 (m, 2H), 7.40(m, 2H), 7.29 (m, 2H), 6.78(s, 1H), 6.70(m, 2H), 6.52(dd, J=8.0, 2.1Hz, 1H), 4.20(m, 4H), 3.00 (dd, *J* = 13.8, 4.4 Hz, 1H), 2.78 (dd, *J* = 13.8, 10.5 Hz, 1H), 1.57(s, 6H). ¹³CNMR δ 173.38, 155.96, 146.69, 145.42, 143.75(2C), 140.70, 140.67, 131.04, 127.62(2C), 127.08, 127.05, 125.31, 125.23, 121.66, 120.10(2C), 117.56, 109.29, 107.73, 65.65, 55.76, 46.60, 36.20, 25.51(2C).

Chemical 8: HRMS [M+H]⁺ Calcd. 474.1911, Found 474.1912. ¹HNMR (400 MHz, DMSO-d*₆*) δ 7.89-7.84 (m, 2H), 7.64 (m, 2H), 7.43-7.39(m, 2H), 7.34-7.27(m,2H), 6.765(d, J= 1.7Hz, 1H), 6.698 (d, J=3.9Hz, 1H), 6.647(dd, J=3.9, 1.7Hz, 1H), 4.242-4.180(m, 4H), 3.62 (s, 3H), 2.954(dd, J=13.8, 4.9Hz, 1H), 2.791(dd, J=13.8, 10.4Hz, 1H), 1.577(s, 6H). ¹³CNMR δ 172.35, 155.89, 146.71, 145.48, 143.70, 140.70, 140.67, 130.53, 127.61, 127.04, 125.22, 125.16, 121.65, 120.10, 117.60, 109.25, 107.75, 65.63, 55.70, 51.90, 36.11, 25.48.

### Example 2: Synthesis of Fmoc-DOPA(cyclohexanonide)-OH as follows

*α.* An amount of 14.3g Na₂B₄O₇·10H₂O (37.5mmol), 200ml water and a magnetic stirring bar were added into a 1000ml three-neck flask. After passing argon for 30min, 14.8g L-DOPA (75mmol) and 8.0g (75mmol) Na₂CO₃ were added, followed by addition of Fmoc-OSu (27.8g, 90mmol) in 200 ml THF. After stirring for 12 hours, the solution was adjusted to pH = 3 with 2N HCl solution, followed by addition of 10-20g Na₂S₂O₃. The mixture was reduced with rotary evaporation, and then extracted with EtOAc. The organic layer was washed with water, dried over anhydrous magnesium sulfate. After filtration, the filtrate was reduced to a small amount with rotary evaporation, followed by addition of petroleum ether to give chemical **5** (white powder, 28.9g, 91%).

*b.* To a 100ml two-neck flask, were added 2.1g (5mmol) of chemical **5**, 5ml of anhydrous acetone and 70ml of anhydrous benzene. After heating and refluxing under argon for 15 minutes, 1.5ml (12.5mmol) of DMP and 20mg TsOH were added. The byproducts H2O / MeOH generated in the reaction system were removed with anhydrous CaCl2 (filled in a Soxhlet extractor or a constant-pressure dropping funnel with fritted glass). The reaction process was monitored with ferric chloride test, and it took about 1-2h to complete. After cooling, the reaction mixture was filtered through a short silica-gel column, which was washed with DCM / EtOAc. The combined filtrate was subjected to rotary evaporation to give a light-yellow solid, which was recrystallized in EtOAc / petroleum ether to produce target chemical **4** with a little amount of **6** (1.5g, 67%).

### Example 3: Synthesis of Fmoc-DOPA(cyclohexanonide)-OH as follows

*α.* An amount of 14.3g Na₂B₄O₇·10H₂O (37.5mmol), 200ml water and a magnetic stirring bar were added into a 1000ml three-neck flask. After passing argon for 30min, 14.8g L-DOPA (75mmol) and 8.0g (75mmol) Na₂CO₃ were added, followed by addition of Fmoc-OSu (27.8g, 90mmol) in 200 ml THF. After stirring for 12 hours, the solution was adjusted to pH = 3 with 2N HCl solution, followed by addition of 10-20g Na₂S₂O₃. The mixture was reduced with rotary evaporation, and then extracted with EtOAc. The organic layer was washed with water, dried over anhydrous magnesium sulfate. After filtration, the filtrate was reduced to a small amount with rotary evaporation, followed by addition of petroleum ether to give chemical **5** (white powder, 28.9g, 91%).

*b.* To a 100ml two-neck flask, were added 2.1g (5mmol) of chemical **5**, 5ml of anhydrous acetone and 70ml of anhydrous benzene. After heating and refluxing under argon for 15 minutes, 1.5ml (12.5mmol) of DMP and 20mg TsOH were added. The byproducts H2O / MeOH generated in the reaction system were removed with anhydrous CaCl2 (filled in a Soxhlet extractor or a constant-pressure dropping funnel with fritted glass). The reaction process was monitored with ferric chloride test, and it took about 1-2h to complete. After cooling, the reaction mixture was filtered through a short silica-gel column, which was washed with DCM / EtOAc. The combined filtrate was subjected to rotary evaporation to give a light-yellow solid, which was recrystallized in EtOAc / petroleum ether to produce target chemical **4** with a little amount of **6** (1.0g, 45%).

### Example 4: Synthesis of Fmoc-DOPA(cyclohexanonide)-OH as follows

*a.* An amount of 14.3g Na₂B₄O₇·10H₂O (37.5mmol), 200ml water and a magnetic stirring bar were added into a 1000ml three-neck flask. After passing argon for 30min, 14.8g L-DOPA (75mmol) and 8.0g (75mmol) Na₂CO₃ were added, followed by addition of Fmoc-OSu (27.8g, 90mmol) in 200 ml THF. After stirring for 12 hours, the solution was adjusted to pH = 3 with 2N HCl solution, followed by addition of 10-20g Na₂S₂O₃. The mixture was reduced with rotary evaporation, and then extracted with EtOAc. The organic layer was washed with water, dried over anhydrous magnesium sulfate. After filtration, the filtrate was reduced to a small amount with rotary evaporation, followed by addition of petroleum ether to give chemical **5** (white powder, 28.9g, 91%).

*b.* To a 100ml two-neck flask, were added 2.1g (5mmol) of chemical 5, 5ml of anhydrous acetone and 70ml of anhydrous benzene. After heating and refluxing under argon for 15 minutes, 1.5ml (12.5mmol) of DMP and 20mg TsOH were added. The byproducts H2O / MeOH generated in the reaction system were removed with anhydrous CaCl2 (filled in a Soxhlet extractor or a constant-pressure dropping funnel with fritted glass). The reaction process was monitored with ferric chloride test, and it took about 1-2h to complete. After cooling, the reaction mixture was filtered through a short silica-gel column, which was washed with DCM / EtOAc. The combined filtrate was subjected to rotary evaporation to give a light-yellow solid, which was recrystallized in EtOAc / petroleum ether to produce target chemical **4** with a little amount of **6** (1.9g, 80%).

### Example 5: Synthesis of Fmoc-DOPA(cyclohexanonide)-OH as follows

*a.* An amount of 14.3g Na₂B₄O₇·10H₂O (37.5mmol), 200ml water and a magnetic stirring bar were added into a 1000ml three-neck flask. After passing argon for 30min, 14.8g L-DOPA (75mmol) and 8.0g (75mmol) Na₂CO₃ were added, followed by addition of Fmoc-OSu (27.8g, 90mmol) in 200 ml THF. After stirring for 12 hours, the solution was adjusted to pH = 3 with 2N HCl solution, followed by addition of 10-20g Na₂S₂O₃. The mixture was reduced with rotary evaporation, and then extracted with EtOAc. The organic layer was washed with water, dried over anhydrous magnesium sulfate. After filtration, the filtrate was reduced to a small amount with rotary evaporation, followed by addition of petroleum ether to give chemical **5** (white powder, 28.9g, 91%).

*b.* To a 100ml two-neck flask, were added 2.1g (5mmol) of chemical **5**, 5ml of anhydrous acetone and 70ml of anhydrous benzene. After heating and refluxing under argon for 15 minutes, 1.55ml (12.5mmol) of DMP and 20mg CSA were added. The byproducts H2O / MeOH generated in the reaction system were removed with anhydrous CaCl2 (filled in a Soxhlet extractor or a constant-pressure dropping funnel with fritted glass). The reaction process was monitored with ferric chloride test, and it took about 1-2h to complete. After cooling, the reaction mixture was filtered through a short silica-gel column, which was washed with DCM / EtOAc. The combined filtrate was subjected to rotary evaporation to give a light-yellow solid, which was recrystallized in EtOAc / petroleum ether to produce target chemical **4** with a little amount of **6** (1.8g, 85%).

### Example 6: Synthesis of L-DOPA and fatty acid conjugate (Lauric acid & L-DOPA)

### The synthesis process is shown in the figure below.

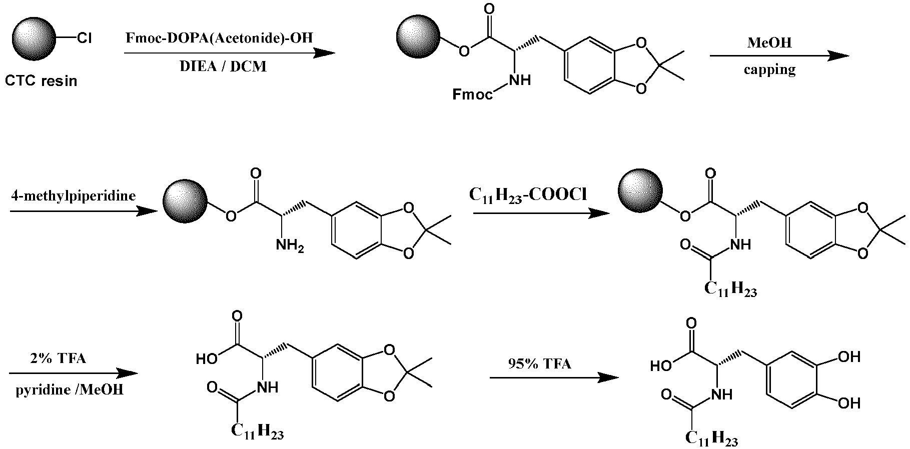

a) An amount of 5g CTC resin was added to a solution of SOCl₂ / DCM (33ml, 1:10) for activation for 5-16h, and then the resin was washed with DCM for 5 times. Fmoc-DOPA(Acetonide)-OH (4.14 g, 9 mmol) was dissolved in DCM, followed by addition of 6.6ml DIEA. The CTC resin was transferred into a SPPS tube and the activated amino acid solution was poured in.

b) The resin was then capped with DIEA / MeOH / DCM (5:15:80), and then the Fmoc was removed with 20% 4-methylpiperidine / DMF. 1.24 ml of lauryl chloride was dissolved in an appropriate amount of DCM, followed by addition of 1.29 ml DIEA. The mixture was poured into the SPPS tube and was shaken for 5-16h. After the reaction was complete as shown by the ninhydrin test, the resin was eluted with a solution of 2% TFA / DCM to obtain the conjugate of L-DOPA and lauric acid in an protected form.

c) When in need, acetonide was subjected to deprotection with a solution of TFA / TIS / H₂O (95 / 2.5 / 2.5) and conjugate FD-12 was obtained. HRMS [M + H]⁺, Cald. 380.2431, Found 380.2435.

### Example 7: Synthesis of L-DOPA and fatty acid conjugate (Stearic acid & L-DOPA)

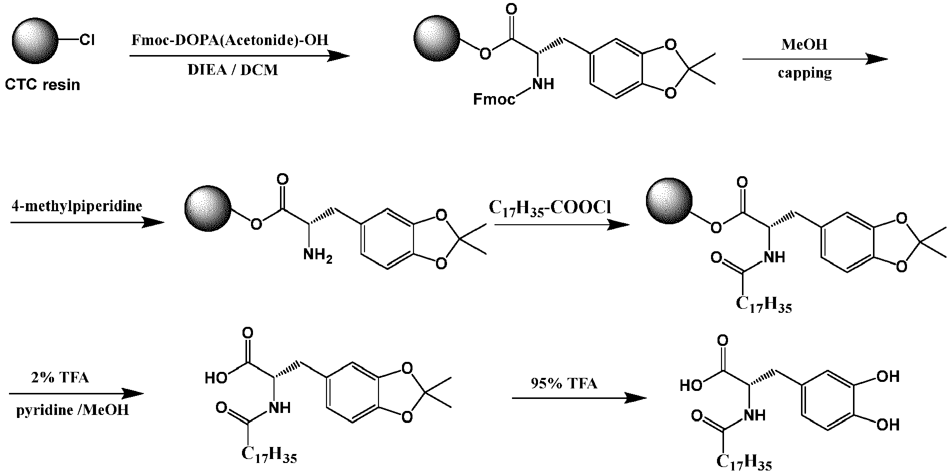

a) An amount of 5g CTC resin was added to a solution of SOCl₂ / DCM (33ml, 1:10) for activation for 5-16h, and then the resin was washed with DCM for 5 times. Fmoc-DOPA(Acetonide)-OH (4.14 g, 9 mmol) was dissolved in DCM, followed by addition of 6.6ml DIEA. The CTC resin was transferred into a SPPS tube and the activated amino acid solution was poured in.

b) The resin was then capped with DIEA / MeOH / DCM (5:15:80), and then the Fmoc was removed with 20% 4-methylpiperidine / DMF. 1.24 ml of stearyl chloride was dissolved in an appropriate amount of DCM, followed by addition of 1.29 ml DIEA. The mixture was poured into the SPPS tube and was shaken for 5-16h. After the reaction was complete as shown by the ninhydrin test, the resin was eluted with a solution of 2% TFA / DCM to obtain the conjugate of L-DOPA and stearic acid in the protected form.

c) When in need, acetonide was subjected to deprotection with a solution of TFA / TIS / H₂O (95 / 2.5 / 2.5) and conjugate FD-18 was obtained. HRMS [M + H]⁺, Cald. 464.3371, Found 464.3373.

**Example 8**: Synthesis of L-DOPA and fatty acid conjugate (Lauric acid & L-DOPA-L-Asp)

The target product was prepared by Fmoc-based SPPS, and the synthesis process is shown in the figure below.

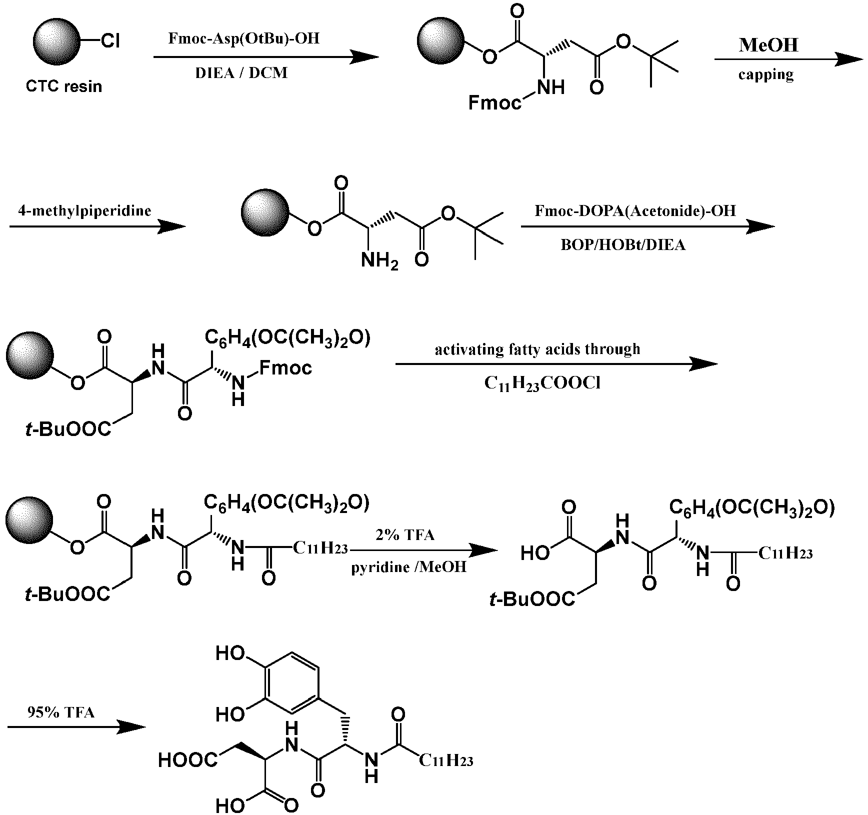

In general, CTC resin was used as a solid-supporting material, Fmoc-DOPA(Acetonide)-OH and Fmoc-Asp(OtBu)-OH as reactants, 4-methylpiperidine as the Fmoc- deprotecting reagent, and lauryl chloride as the activation form of lauric acid. The synthesized conjugate of fatty acid and L-DOPA-containing dipeptide was cut off from the resin with a solution of 2% TFA to give the intermediate conjugate with the side chain protected.

The target conjugate of fatty acid and L-DOPA-containing dipeptide was obtained by rapidly removing the side chain protection with 95% TFA. FDD-12 was characterized by HRMS [M + H]⁺, Calcd. 495.2700, Found 495.2701.

### Example 9: Synthesis of L-DOPA and fatty acid conjugate (palmitic acid & L-DOPA-L-Asp)

The target product was prepared using the Fmoc-based SPPS protocol, and the synthesis process is shown in the figure below.

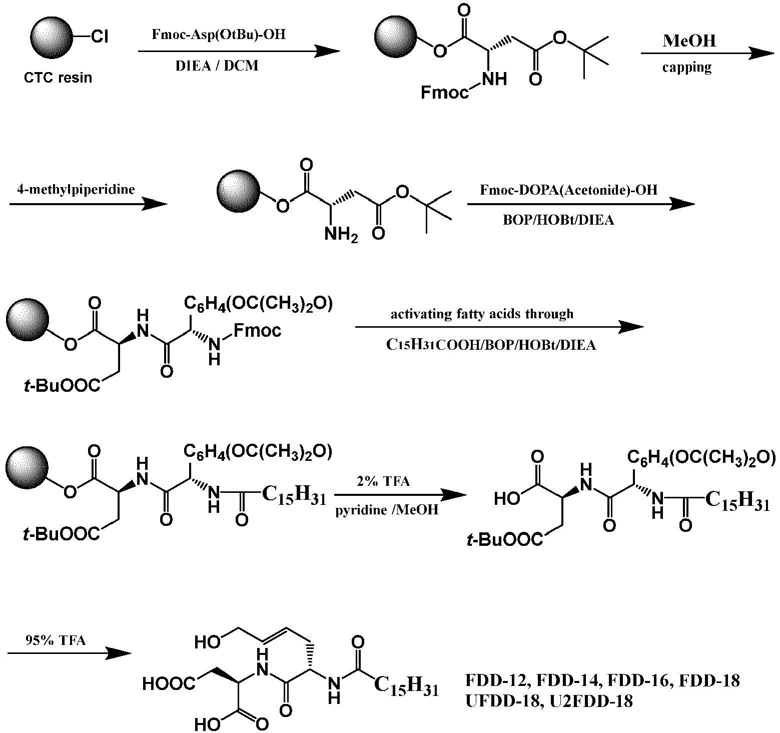

CTC resin was used as a solid-supporting material; Fmoc-DOPA(Acetonide)-OH and Fmoc-Asp(OtBu)-OH were used as reactants; 4-methylpiperidine was used as Fmoc deprotecting reagent; and palmitic acid was activated with BOP / HOBt / DIEA. The synthesized fatty acid complex containing L-DOPA dipeptide was cut off from the resin with 2% TFA to give the intermediate conjugate with the side chain protected.

The target conjugate of fatty acid and L-DOPA dipeptide was obtained by rapidly removing the side chain protection of the intermediate with 95% TFA. The target conjugate FDD-16 was characterized by HRMS, [M + H]⁺, Calcd. 551.3317, Found 551.3327.

### Example 10: Synthesis of L-DOPA-containing dipeptide and fatty acid conjugate (tetradecanoic acid & L-DOPA-L-Asp)

The target product was prepared using the Fmoc-based SPPS protocol, and the synthesis process is shown in the figure below.

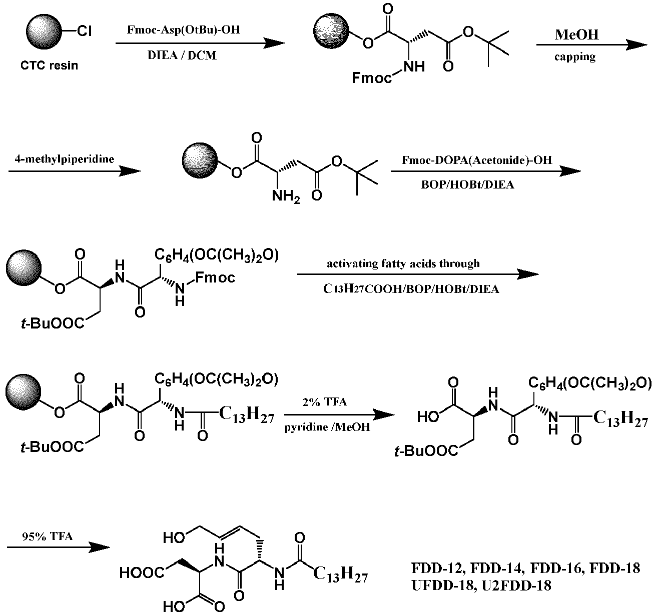

CTC resin was used as a solid-supporting material; Fmoc-DOPA(Acetonide)-OH and Fmoc-Asp(OtBu)-OH were used as reactants; 4-methylpiperidine was used as Fmoc deprotecting reagent; and tetradecanoic acid was activated with BOP / HOBt / DIEA. The synthesized fatty acid complex containing L-DOPA dipeptide was cut off from the resin with 2% TFA to give the intermediate conjugate with the side chain protected.

The target conjugate of fatty acid and L-DOPA dipeptide was obtained by rapidly removing the side chain protection of the intermediate with 95% TFA. The target conjugate FDD-14 was characterized by HRMS, [M + H]⁺, Calcd. 523.3014, Found 523.3015.

### Example 11: Synthesis of L-DOPA and fatty acid conjugate (stearic acid & L-DOPA-L-Asp)

The target product was prepared using the Fmoc-based SPPS protocol, and the synthesis process is shown in the figure below.

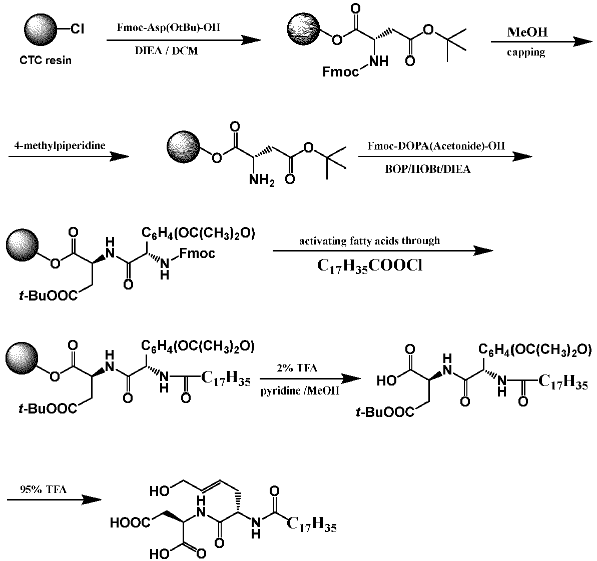

CTC resin was used as a solid-supporting material; Fmoc-DOPA(Acetonide)-OH and Fmoc-Asp(OtBu)-OH were used as reactants; 4-methylpiperidine was used as Fmoc deprotecting reagent; and stearyl chloride was used as the activated fatty acid form. The synthesized fatty acid complex containing L-DOPA dipeptide was cut off from the resin with 2% TFA to give the intermediate conjugate with the side chain protected.

The target conjugate of fatty acid and L-DOPA dipeptide was obtained by rapidly removing the side chain protection of the intermediate with 95% TFA. The target conjugate FDD-18 was characterized by HRMS, [M + H]⁺, Calcd. 579.3640, Found 579.3635.

### Example 12: Synthesis of L-DOPA-containing dipeptide and fatty acid conjugate (oleic acid & L-DOPA-L-Asp)

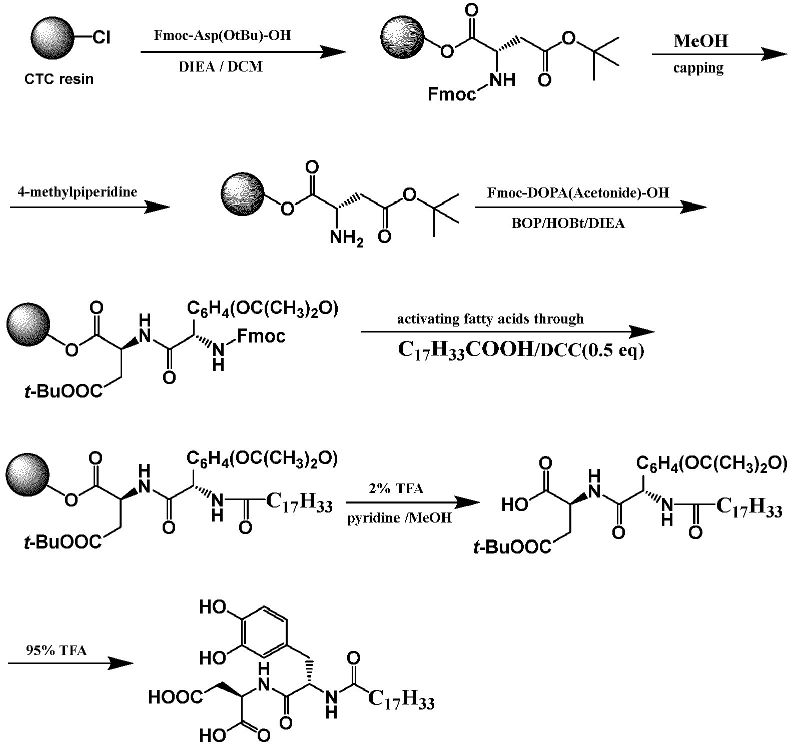

The target product was prepared using the Fmoc-based SPPS protocol, and the synthesis process was like that shown in example 7. In short, CTC resin was used as a solid-supporting material; intermediates Fmoc-DOPA(Acetonide)-OH and Fmoc-Asp(OtBu)-OH were used as reactants; 4-methylpiperidine was used as Fmoc deprotecting reagent; and oleic acid was activated with DCC in the form of anhydride. The synthesized fatty acid complex containing L-DOPA dipeptide was cut off from the resin with 2% TFA to give the intermediate conjugate with the side chain protected.

The target conjugate of fatty acid and L-DOPA dipeptide was obtained by rapidly removing the side chain protection of the intermediate with 95% TFA. The target conjugate UFDD-18 was characterized by HRMS, [M + H]⁺, Calcd. 563.3460, Found 563.3435.

### Validation Embodiments:

**Part** I. Gel formation tests of fatty acid conjugates with L-DOPA-containing dipeptides or L-DOPA in organic solvents.

### 1. Gel formation tests of L-DOPA-containing lipodipeptides

### 1.1 Gel formation test of FDD-16 and searching for proper concentrations

1) Six samples of 40 mg FDD-16 was added into six 2ml centrifuge tubes, followed by addition of 1 ml of respective organic solvents. Mother solutions were prepared using MeOH, ethanol, toluene, THF, DMSO, and DMF, respectively.

2) Aliquots of 50 µl of the above mother solutions were added, respectively, to nine 2ml centrifuge tubes, followed by addition of organic solvents (MeOH, ethanol, toluene, THF, DMSO, DMF, respectively). The volume ratios of the organic solvent to distilled water were 1:9, 2:8, 3:7, 4:6, 5:5, 6:4, 7:3, 8:2 and 9:1, respectively. Tests of each solvent ratio were repeated 3 times. Gel formation rates and the corresponding sample concentrations were marked down.

### 1.2 Gel formation tests for other lipodipeptides and searching for proper concentrations

The experiments were performed like those of FDD-16.

### 1.3 Experimental results

The results showed that FDD-12 could not form gels, and it could be deduced that fatty acids with less than 12 C could not form gels under the above conditions; conjugates FDD-16, FDD-18 and FDD-14 could form stable gels. FDD-16 and FDD-14 had a wide range of gelation concentrations. The details are as follows:

### Table 1 Gelation tests of L-DOPA -containing lipodipeptides (I)

| | MeOH | | Ethanol | | Toluene | |
|---|---|---|---|---|---|---|
| Samples | Gelation concentration (g/mL) | Volume ratio to water (V:V) | Gelation concentration (g/mL) | Volume ratio to water (V:V) | Gelation concentration (g/mL) | Volume ratio to water (V:V) |
| FDD-12 | - | - | - | - | - | - |
| FDD-16 | 6~26 | 15/85~65/35 | 6~24 | 15/85~60/40 | - | - |
| FDD-18 | 10~16 | 25/75~40/60 | 12~26 | 30/70~65/35 | - | - |
| FDD-14 | 8~22 | 20/80~55/45 | 8~20 | 20/80~50/50 | - | - |
| UFDD-18 | - | - | - | - | - | - |
| U2FDD18 | - | - | - | - | - | - |

### Table 2 Gelation tests of L-DOPA -containing lipodipeptides (II)

| | THF | | DMSO | | DMF | |
|---|---|---|---|---|---|---|
| Samples | Gelation concentration (g/mL) | Volume ratio to water (V:V) | Gelation concentrati on (g/mL) | Volume ratio to water (V:V) | Gelation concentration (g/mL) | Volume ratio to water (V:V) |
| FDD-12 | - | - | - | - | - | - |
| FDD-16 | 8~12 | 20/80~30/70 | 6~28 | 15/85~70/30 | 6~22 | 15/85~55/45 |
| FDD-18 | - | - | 16~30 | 40/60~75/25 | 14~26 | 35/65~65/35 |
| FDD-14 | 6~8 | 15/85~20/80 | 8~22 | 20/80~55/45 | 6~20 | 15/85~50/50 |
| UFDD-18 | - | - | - | - | - | - |
| U2FDD18 | - | - | - | - | - | - |

The results from Table 2 and 3 demonstrated that UFDD-18 and U2FDD18 could not form gels in organic solvents (MeOH, ethanol, toluene, THF, DMSO, DMF), but it did not exclude the possibility of gelling in other organic solvents in subsequent development. FDD-16 and FDD-14 formed gels in MeOH, ethanol, THF, DMSO and DMF, and FDD-18 formed gels in MeOH, ethanol, DMSO and DMF. In terms of the organic solvents selected in this invention, the range of gelling concentrations of FDD-18 is narrower than those of FDD-16 and FDD-14.

### 2. Gelation tests of conjugates of fatty acid and L-DOPA

### Gel formation experiments on FD-12 and FD-12

Tests were performed according to the tests done for FDD-16.

**Table 3 Gelation tests of L-DOPA -containing lipodipeptides (II)**

| MeOH | | Ethanol | | | Toluene | |
|---|---|---|---|---|---|---|
| Samples | Gelation concentration (g/mL) | Volume ratio to water (V:V) | Gelation concentration (g/mL) | Volume ratio to water (V:V) | Gelation concentration (g/mL) | Volume ratio to water (V:V) |
| FD-12 | - | - | - | - | - | - |
| FD-18 | 10~15 | 25/85~55/25- | 10~14 | 35/75~60/40 | - | - |

**Table 4 Gelation tests of L-DOPA -containing lipodipeptides (II)**

| | THF | | DMSO | | DMF | |
|---|---|---|---|---|---|---|
| Samples | Gelation concentration (g/mL) | Volume ratio to water (V:V) | Gelation concentratio n (g/mL) | Volume ratio to water (V:V) | Gelation concentration (g/mL) | Volume ratio to water (V:V) |
| FDD-12 | - | - | - | - | - | - |
| U2FDD18 | 10~16 | 26/50~40/60 | 16~28 | 45/85~70/50 | 16~24 | 45/85~55/35 |

### Part 2. Characterization of gels formed from FDD-16

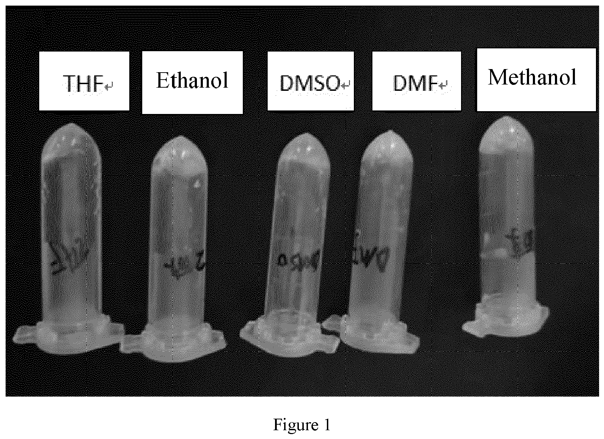

The appearance and stability of gels formed from FDD-16 was studied by turning the tubes upside down and . The results showed that the gel formed from FDD-16 was in good condition and remained at the bottom while standing for long time. This shed lights on future druggability study of FDD-16 using MeOH, ethanol, THF, DMSO and DMF as solvents. See figure 1 for gel formation experiments.

### Part 3. Scanning Electron Microscopy (SEM) images of gels formed from FDD-18 or FDD-14

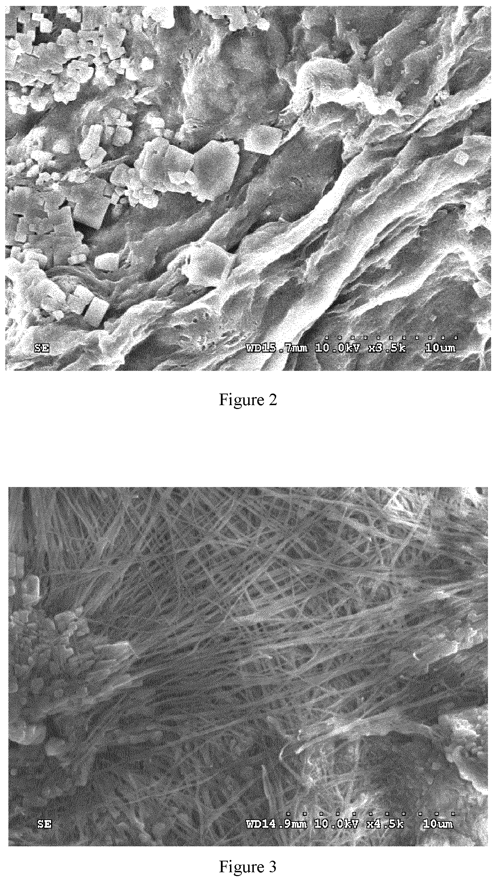

Through the gelling experiments, it was found that FDD-18 and FDD-14 exhibited higher possibilities to form gels. The images of electron microscope observation on gels formed from FDD-18 or FDD-14 were shown in Figure 2 and Figure 3, respectively.

## Claims

1. A synthetic method for intermediates to make L-DOPA oligopeptides is **characterized in that** compound I is obtained by catalytic condensation of a cyclizing reagent with starting compound II in the presence of a strong acid *b* and an organic cosolvent *a.* The reaction equation is as follows.

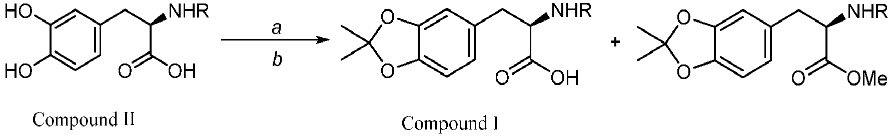

2. The synthetic method according to claim 1 is **characterized in that** it comprises the following steps: adding organic cosolvent *a* and compound II to the reaction system, heating and refluxing under the protection of an inert gas, adding a cyclizing reagent and strong acid b, adding adsorbents to adsorb water and volatile byproducts, and finally, compound I Fmoc-DOPA-(Acetonide)-OH, was obtained.

3. The synthetic method according to claim 2 is **characterized in that** the organic cosolvent a is an anhydrous solvent, including but not limited to acetone, benzene, toluene, xylene, ether, methyl ethyl ketone, methyl isobutyl ketone and pyridine, preferably a mixture of anhydrous acetone and anhydrous benzene; The strong acid b is one or a combination of TsOH, camphorsulfonic acid, benzoic acid, hydrogen halides, TFA, acetic acid and strong acid ion exchange resins, preferably TsOH; The cyclizing reagent is one or a combination of ketone or aldehyde derivatives, such as acetone, 2,2-dimethoxypropane, 2-methoxypropylene, cyclopentanone, cyclohexanone and benzophenone, preferably dimethoxypropane or cyclohexanone; The R group is an amino protecting group, including but not limited to Phth-, TCP-, DTS-, Tfa-, Ac-, Aloc-, MeCO₂-, EtCO₂-, Boc-, Fmoc-, Teoc -, Troc-, SES-, Tr-, preferably Fmoc-.

4. The synthetic method according to claim 2 is **characterized in that** the absorbent is anhydrous calcium chloride, anhydrous magnesium sulfate, anhydrous sodium sulfate and molecular sieves; Anhydrous calcium chloride is preferred.

5. The synthetic method according to claim 2 is **characterized in that** the molar ratio of compound II to DMP is 0.8-20, preferably 2.5; The molar ratio of compound II to cyclohexanone is 1-50, preferably 10; The reflux temperature of the solvent is 80 ~ 120°C.

6. New intermediates or final products synthesized according to any one of claims 1 to 5, wherein compound I is obtained according to any synthetic method of claims 1 to 5.

7. A fatty acid conjugate containing DOPA oligopeptides, especially dipeptides; The structural formula of the fatty acid conjugate is as follows. 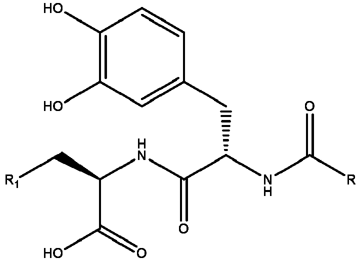
(1) Synthesized from compound I according to claim 1, which is obtained by the synthetic method according to any one of claims 1-5;
(2) Wherein, R represents the chain of a linear or branched fatty acid of C12 ~ C30, including saturated or unsaturated, preferably one of lauric acid, palmitic acid, tetradecanoic acid, lauric acid, stearic acid, linoleic acid, oleic acid, linolenic acid and docosahexaenoic acid, more preferably one of palmitic acid, stearic acid and tetradecanoic acid; R₁ is an L-type or D-type amino acid side-chain, including but not limited to any of 20 common amino acids and one of beta alanine, taurine and citrulline; More preferably aspartic acid; L-DOPA is located at the *N*-terminal or *C*-terminal.

8. Applications of compound I obtained by the synthetic method according to any one of claims 1-5 in the preparations of fatty acid derivatives of L-DOPA, fatty acid conjugates of L-DOPA, and sustained-release prodrugs against Parkinson's disease.

9. A pharmaceutical composition for simultaneously supplementing nutrition and providing DOPA medicine for Parkinson's patients, which is **characterized in that** the pharmaceutical composition comprises one or more pharmaceutically acceptable excipients and one or more compounds selected from the following chemicals: compound I obtained by the synthetic method according to any one of claims 1-5, the fatty acid complexes according to claim 7, the intermediate products or end products synthesized with compound I obtained using the synthetic method according to any one of claims 1-5, and pharmaceutically acceptable salts thereof.

10. A preparation of anti-Parkinson's disease prodrugs formed by the following compounds: L-DOPA-containing oligopeptides, lipid oligopeptides obtained according to the synthetic method of any one of claims 1-5, fatty acid complexes obtained according to claim 7, the intermediate products or end products synthesized with compound I according to any one of claims 1-5, a pharmaceutically acceptable salt thereof; with or without medicinal polymer materials. Thereinto, L-DOPA metabolic enzyme inhibitors are added or not added; Moreover, the preparation is further selected as a gel, an injection, or an oral agent.
